# EUROPÄISCHE PATENTSCHRIFT

(11) **EP 4 747 616 B1**
(45) Veröffentlichungstag und Bekanntmachung des Hinweises auf die Patenterteilung: **29.07.2026**
(21) Anmeldenummer: 25759085.1
(22) Anmeldetag: 19.08.2025
(51) Int. Cl.: G01N 25/48, G01K 19/00, G01K 17/04, G01N 33/38

(54) **KALIBRIERVERFAHREN FÜR EIN WÄRMEFLUSSKALORIMETER, INSBESONDERE IN DER ZEMENTINDUSTRIE**
METHOD FOR CALIBRATING A HEAT FLOW CALORIMETER, IN PARTICULAR IN THE CEMENT INDUSTRY
PROCÉDÉ DE CALIBRAGE D'UN CALORIMÈTRE À FLUX THERMIQUE, EN PARTICULIER DANS L'INDUSTRIE DU CIMENT

(30) Priorität: 29.08.2024 BE 202405576; 29.08.2024 DE 102024124649
(43) Veröffentlichungstag der Anmeldung: 27.05.2026
(73) Patentinhaber: thyssenkrupp Polysius GmbH, 59269 Beckum (DE); thyssenkrupp AG, 45143 Essen (DE)
(72) Erfinder: ENDERS, Michael, 48143 Münster (DE)
(74) Vertreter: thyssenkrupp Intellectual Property GmbH
(86) Internationale Anmeldenummer: PCT/EP2025/073699
(87) Internationale Veröffentlichungsnummer: WO 2026/046783

(56) Entgegenhaltungen:
- WO-A1-2023/094410
- VON MENTZER KRISTINA: "Study on Early Hydration Reactions of Cement Clinker using Isothermal Calorimetry - Influence of C3A content, SO3 content, Blaine Number and Mixing Type", 2 July 2024 (2024-07-02), pages 1 - 70, XP093251775, Retrieved from the Internet <URL:http://lup.lub.lu.se/student-papers/record/9169683> [retrieved on 20250218]
- SANDBERG PAUL ET AL: "Automated calorimetry - the missing link in quality control", INTERNATIONAL CEMENT REVIEW, 30 June 2020 (2020-06-30), XP093250009, Retrieved from the Internet <URL:https://ucpcdn.thyssenkrupp.com/_binary/UCPthyssenkruppBAIS/en/products-and-services/cement-plants/laboratory-automation/polab(r)/thyssenkrupps-polabcal/link-ICR-June-2020-REPRINT-thyssenkrupp-Automated-calorimetry-HR.pdf> [retrieved on 20250218]

## Beschreibung

Die Erfindung betrifft ein Verfahren zur automatischen Kalibrierung eines Wärmeflusskalorimeters, um einen sofortigen Messbeginn mit reproduzierbaren und aussagekräftigen Messwerten auch innerhalb der erste Stunde zu ermöglichen.

Die isotherme Wärmeflusskalorimetrie wird in der Zementforschung angewendet, um das Reaktionsverhalten von Bindemitteln über der Zeit abzubilden. Das isotherme Wärmeflusskalorimeter weist wenigstens eine präzise innerhalb etwa ²/₁₀₀ bis ¹/₁₀₀₀ K temperaturgeregelten Kalorimeterkammer auf und die Kalorimeterkammer wieder weist eine oder mehrere, meist acht, in der Kalorimeterkammer eingebaute Kalorimeterzellen auf. Die Temperatur der Kalorimeterzellen wird über die Kalorimeterkammer stabilisiert. In der Kalorimeterzelle nimmt ein Wärmeflusssensor die aktuell zu einer Wärmesenke abfließende Wärme auf und bildet so die aktuelle Wärmedifferenz zwischen der Probe und der Wärmesenke ab. Die freigesetzte Wärmemenge ist abhängig von der Zusammensetzung des Bindemittels, der Feinheit und eventuellen weiteren Zusätzen. Die kumulative spezifische Wärme der Probe ist eine Materialkonstante, und nur durch eine Änderung von physikalischen Parametern, wie der Feinheit oder der Zusammensetzung oder durch die Zugabe von beispielsweise Betonzusatzmitteln, zum Beispiel Wasserreduzierer, Beschleuniger, Verzögerer und dergleichen, ist eine Änderung der Reaktionskinetik möglich. Typische Experimente sind dann im Bereich der Zementchemie der Einfluss dieser externen Parameter, beispielsweise Feinheit, Zusammensetzung, Zusatzmittel, auf den Reaktionsverlauf. Abgebildet wird der Wärmefluss über der Zeit oder die kumulative Wärme über der Zeit, so dass im Ergebnis die Intensität der Zementhydration über der Zeit abgebildet wird.

Die Probenvorbereitung erfolgt hierbei üblicherweise manuell. Hierzu wird eine Bindemittelprobe zunächst in eine Ampulle eingewogen und anschließend mit einer definierten Wassermenge so versetzt, dass ein ausgewähltes Wasser/Bindemittelverhältnis eingestellt wird (Wasser/Zementwert). Nach der Wasserzugabe werden das Pulver und das Wasser vor Befüllung einer Ampulle oder direkt in der Ampulle manuell mit einem Spatel oder maschinell durch Schütteln oder Vibration zu einer homogenen Paste gemischt. Die Ampulle mit der homogenen Paste wird nach der Homogenisierung mit einem Werkzeug, beispielsweise einem Haken oder einem Sauger, in eine der Kalorimeterzellen in der Kalorimeterkammer eines kommerziell verfügbaren Kalorimeters eingesetzt. Die Messung wird anschließend über die vom Hersteller des Kalorimeters beigestellte Software zur kontinuierlichen Aufnahme der aktuell freigesetzten Wärmemenge gestartet.

In diesem weitverbreiteten Verfahren werden die üblicherweise ersten 30-60 Minuten der Messung nicht berücksichtigt, da mögliche Temperaturunterschiede zwischen der Temperatur innerhalb des isothermen Kalorimeters und der außerhalb des isothermen Kalorimeters vorbereiteten Probe gemeinsam mit dem Messsignal erfasst werden und so zu einem erheblichen Messfehler führen. Nach einer Wartezeit von 30-60 Minuten zeigt die Stabilisierung der base-line an, dass die aus der Temperaturdifferenz entstandene zusätzliche, nicht bei der Hydratation freigesetzte Wärmemenge vollständig abgeführt ist, und die im folgenden freigesetzte Wärmemenge allein der Probe zugeordnet werden kann.

Dieser Effekt ist sehr gut mit einer inerten (Sand) Probe zu zeigen. Diese Probe reagiert bei Zugabe von Wasser oder einer Anregerflüssigkeit nicht und setzt keine Energie aus einer chemischen Reaktion frei. In einem ideal temperierten System sollte also die Probe bei Einschleusen in eine Kalorimeterzelle keine messbare Wärme freisetzen. Ist die Probe nicht auf der gleichen Temperatur wie das Kalorimeter innen, beobachtet man auch für die inerte Probe eine Abweichung von der Nulllinie, nämlich zu negativen Werten, wenn die Probe kälter als die Innentemperatur des Kalorimeters ist, oder zu positiven Werten, wenn die Probe wärmer als die Innentemperatur des Kalorimeters ist. Technisch erklärt werden diese Beobachtungen durch einen Wärmefluss aus der Wärmesenke in die Probe beziehungsweise einen Wärmefluss aus der Probe in die Wärmesenke. Das isotherme Kalorimeter ist also ein ideales Instrument, um an inerten Proben absolute Temperaturunterschiede zu bestimmen.

In einer realen Bindemittelprobe kann jedoch anders als in einer inerten Probe der Wärmebetrag durch Hydratation der Probe und der Wärmebetrag aus einer Temperaturdifferenz zwischen der Probe und dem Thermostaten des Kalorimeters nicht unterschieden werden. Hierdurch entsteht ein systematischer Messfehler dann, wenn Messwerte aus einem Zeitraum berücksichtigt werden, in dem dieser Messfehler noch relevant ist. Allerdings ist bekannt, dass innerhalb von 30-60 min die auf die Probentemperatur zurückgehende Wärmemenge vollständig oder weitgehend über die Wärmesenke abgeführt wird. Also sind vertrauenswürdige Messungen erst nach 30-60 Minuten Wartezeit möglich.

In der Praxis wurden zwei Verfahren vorgeschlagen, um auch die initiale Wärmeabgabe innerhalb der ersten 30-60 min der Reaktion bestimmen zu können. Bei der in-Situ Messung werden in einer spezialisierten Ampulle Pulver und Wasser getrennt in die Kalorimeterzelle eingeführt. Die Temperaturstabilisierung des Bindemittels und des Anmachwassers in getrennten Behältern erfolgt innerhalb der Kalorimeterkammer. Nach einer Temperaturstabilisierung von meist mehreren Stunden, üblicherweise 2 bis 20 h, wird über einen Mechanismus von außen zunächst der Behälter mit dem Wasser geöffnet, und das Wasser fließt zu dem Pulver, und abschließend wird von außen manuell oder mechanisch angetrieben mit einer Rührvorrichtung eine Paste aus Wasser und dem Bindemittel innerhalb der Kalorimeterzelle durch Mischen hergestellt. Die Messung startet ab der Wasserzugabe. Das Verfahren wird nicht in Routinemessungen eingesetzt und ist nicht automatisierbar. Gründe hierfür sind die lange Wartezeit auf Temperaturstabilisierung der einzeln einbrachten Pastenbestandteile im Kalorimeter und damit ein nur geringer Probendurchsatz, die mangels Sichtkontakt nicht verifizierbare Mischqualität und die nur teilweise wiederverwendbaren oder nur aufwendig zu reinigenden spezialisierten Ampullen und Rührer.

WO 2016 / 041 717 A1, WO 2017 / 162 639 A1 und US 10 352 885 B2 nutzen abweichend den Ansatz die gesamte Probenvorbereitung und eine optionale Zwischenlagerung der Probe in einer Probenvorbereitungskammer mit einem direkten Zugang zu dem Kalorimeter durchzuführen. Die Temperaturgleichheit von Probenvorbereitung und Kalorimeterkammer minimiert die Temperaturdifferenz der Proben beim Einschleusen in die Kalorimeterkammer und die isotherme kalorimetrische Messung kann ohne Verzug unmittelbar nach der Probenzufuhr und ohne Warten auf Stabilisierung der base-line beginnen.

In der Praxis wird hierzu eine Probenvorbereitungskammer auf einer konstanten präzisen geregelten Temperatur gehalten. Optional kann zudem ein Probenlager vorgesehen sein, wie zum Beispiel in der WO 2023 / 094 419 vorgeschlagen. Das Probenlager wird mit einem zweiten Temperaturregler stabilisiert, um eine Probentemperaturdifferenz zwischen der Umgebung der temperierten Probenvorbereitung und der Probenvorbereitungskammer schneller auszugleichen. Das an die Probenvorbereitungskammer angeschlossene isotherme Kalorimeter verfügt über einen weiteren eigenen Temperaturregler zur Überwachung und Regelung der Temperatur innerhalb des Kalorimeterkammer mit den Kalorimeterzellen.

Die Temperaturmessung mit Thermoelementen ist sehr gut geeignet, relative Temperaturunterschiede bei Änderung der Umgebungstemperatur zu messen. Schwieriger ist es, absolute Temperaturen präzise mit Thermoelementen zu messen. Dies ist nur dann möglich, wenn die Thermoelemente präzise kalibriert worden sind und die in der Regel vorkommenden variablen Leitungseigenschaften und herstellungsbedingten Abweichungen ausgeglichen werden. Aus diesem Grund wird für Thermoelemente häufig zum Beispiel in Eiswasser (0 °C) oder kochendem Wasser (100 °C) der absolute offset bestimmt und dann die Temperaturmessung mit einem spezifischen Korrekturwert korrigiert.

Eine weitere Schwierigkeit ist insbesondere bei größeren Volumen, dass die Temperatur am Einbauort des Thermoelements zum Beispiel durch eine vertikale Temperaturschichtung eine abweichende Temperatur gegen den am Regler vorgegebenen Stellwert anzeigt, der die in das Volumen eingebrachte Temperatur inklusive des Anzeigefehlers des Thermoelements einstellt. Zuletzt können sich auch durch dynamische Luftströmungen innerhalb eines Volumens Temperaturdifferenzen auch in einer in sich geschlossenen Zelle ergeben.

Das Verfahren nach WO 2016 / 041 717 A1, WO 2017 / 162 639 A1 oder US 10 352 885 B2 erfordert bei der Erstinbetriebnahme und gegebenenfalls im laufenden Betrieb einen Abgleich der Temperaturen in der Probenvorbereitungskammer mit der Innentemperatur des Kalorimeters (Kalorimeterkammer), um dauerhaft über die Temperaturgleichheit belastbare Messungen ohne eine Fehlbestimmung aufgrund einer Temperaturdifferenz zwischen der Probe und dem isothermen Kalorimeter sicherzustellen. Eine direkte Kalibration der Thermoelemente relativ zueinander ist aufgrund der Einbausituation in Geräten nicht möglich. Aus diesem Grunde erfolgt der Abgleich der Probenpräparationskammertemperatur und der Temperatur im Kalorimeters mit Hilfe einer inerten Probe, um Temperaturdifferenzen zwischen der Probenpräparationskammer und dem Kalorimeterinneren zu bestimmen und dann die Reglereinstellung so anzupassen, dass die Temperaturunterschiede gegen null gehen.

Hierzu kann man zum Beispiel die inerten Proben bei variabler Temperatur in das Kalorimeter einschleusen und eine Korrelation des Messsignals mit der Probentemperatur erstellen. Aus der Korrelation kann man dann über die beobachtete Temperaturdifferenz die notwendige Änderung an den Temperaturreglern abschätzen und iterativ optimieren. In der Praxis wird die Temperatur des Thermostaten des Kalorimeters als Festpunkt genommen und in wiederholten Messungen zunächst die Reglereinstellung für die Heiz/Kühleinrichtung der Probenpräparationskammer so verändert, dass beim erneuten Einschleusen der inerten Probe das Messsignal in Richtung der Nulllinie verändert wird.

Der Endpunkt ist erreicht, wenn für die inerte Probe kein Messsignal oder eir vernachlässigbares Messsignal mit dem isothermen Kalorimeter festgestellt werden kann.

Aufwendiger - insbesondere aufgrund von Wartezeiten zeitaufwendiger - wird es, wenn in eine gemeinsame Probenpräparation eine zweite oder mehrere Kalorimeterkammern angeschlossen sind. In diesem Fall liegt anfangs in der Regel sowohl eine bedingt durch mehrere Thermoelemente abweichende Innentemperatur der Kalorimeterkammern vor und eine abweichende Temperatur der Probenvorbereitungskammer. In diesem Fall muss für alle weiteren Kalorimeterkammern auch der Abgleich mit dem ersten Kalorimeterkammer durchgeführt werden, so dass zuletzt eine inerte Probe unabhängig vom Messort in der ersten oder einer der weiteren Kalorimeterkammern ein korrektes Messsignal auf and nahe der Nulllinie ermittelt wird. Es ist offensichtlich, dass mit steigender Anzahl an Kalorimeterkammern der Abgleich der Stellpunkte (setpoints) aufwendiger wird. Die Temperaturangleichung der Kalorimeterkammern ist unabdingbar, da andernfalls aufgrund der veränderten Reaktionskinetik durch die ungleichen Innentemperaturen keine vergleichbaren Messungen in verschiedenen Kalorimeterkammern durchgeführt werden können.

Aus der Von Mentzer Kristina: "Study on Early Hydration Reactions of Cement Clinker using Isothermal Calorimetry - Influence of C3A content, S03 content, Blaine Number and Mixing Type", 2. Juli 2024, XP093251775, Gefunden im Internet: URL:http://lup.lub.lu.se/student-papers/record/9169683 ist eine wissenschaftliche Studie zur Hydration von Klinker bekannt.

Aus der WO 2023/094410 A1 ist eine Anlage mit einem Probenlager zur Vorbereitung eines Probenmaterials der Zement- oder Kalkindustrie auf eine kalorimetrische Messung bekannt.

Aus der Sandberg Paul ET AL: "Automated calorimetry - the missing link in quality control", International Cement Review, 30. Juni 2020, XP093250009, Gefunden im Internet: URL:https://ucpcdn.thyssenkrupp.com/_binary/UCPthyssenkruppBAIS/en/products-andservices/cement-plants/laboratory-automation/polab(r)/thyssenkrupps-polabcal/link-ICR-June-2020-REPRINT-thyssenkruppAutomated-calorimetry-H R.pdf ist eine Übersicht zur automatisierten Kalorimetrie bekannt.

Aufgabe der Erfindung ist es, ein automatisiertes Regelsystem zu schaffen, das reale Temperaturdifferenzen an unterschiedlichen Bestandteilen in einem automatisierten Kalorimeter erfasst, und die Controller der Kühl/Heizaggregate trotz abweichender Temperaturanzeigen in einer automatisierten Prozedur so regelt, dass ein oder mehrere Kalorimeter und die Probenpräparationskammer auf der gleichen oder einer hinlänglich gleichen Temperatur dauerhaft ohne Messfehler betrieben werden können und der Aufwand des Temperaturabgleichs klein wird.

Gelöst wird diese Aufgabe durch das Verfahren mit den in Anspruch 1 angegebenen Merkmalen. Vorteilhafte Weiterbildungen ergeben sich aus den Unteransprüchen, der nachfolgenden Beschreibung sowie den Zeichnungen.

Das erfindungsgemäße Verfahren dient der Kalibrierung eines Wärmeflusskalorimeters mit wenigstens einer Probenpräparationskammer und wenigstens einer ersten Kalorimeterkammer. Hierbei ist die Kalorimeterkammer der Ort, an dem die tatsächliche Messung des Wärmeflusses durchgeführt wird. Industriell eingesetzte Kalorimeter haben oftmals acht oder mehr parallel betreibbare Kalorimeterzellen in einer Kalorimeterkammer, welche alle gleichwertig sein können. Die Auswahl der ersten Kalorimeterkammer aus der Gesamtheit kann dann beliebig definiert werden. Gleichzeitig kann die Kalorimeterkammer zwei oder mehr Kalorimeterzellen enthalten, wobei jede Kalorimeterzelle zwar eine eigenständige Messung als Kalorimeter vornehmen kann, aber alle Kalorimeterzellen innerhalb der Kalorimeterkammer gemeinsam über nur eine gemeinsame Temperierung temperiert werden. Daher sind die Kalorimeterzellen für das erfindungsgemäße Verfahren nicht unterscheidbar und gleichwertig. Die Probenpräparationskammer weist eine Präparationskammertemperierung und ein Präparationskammerthermoelement auf. Die erste Kalorimeterkammer weist eine erste Kalorimetertemperierung und ein erstes Kalorimeterthermoelement auf. Das Thermoelement dient hierbei jeweils der Erfassung der Temperatur, nach der dann die Temperierung geregelt wird. Da aber mit einem Thermoelement, wie bereits ausgeführt, absolute Temperaturen schwer zu messen sind, ergibt sich zwischen den verschiedenen Thermoelementen ein Unterschied bei einer gleichen erfassten Temperatur sodass die verschiedenen Temperierungen auf leicht unterschiedliche Temperaturen geregelt werden, was wiederum dazu führt, das eine Probe, die von einem in den anderen Bereich transportiert wird, eine andere Temperatur aufweist als der Bereich, in den diese transportiert wird, was wiederum eine Messung eine Messung des Wärmeflusses im Kalorimeter verfälscht. Das Wärmeflusskalorimeter weist eine Steuereinheit auf. Die Steuereinheit ist mit dem Präparationskammerthermoelement und dem ersten Kalorimeterthermoelement zur Übertragung der erfassten Temperaturen an die Steuereinheit verbunden. Weiter ist die Steuereinheit mit der Präparationskammertemperierung und der ersten Kalorimetertemperierung zur Ansteuerung verbunden. Das Verfahren weist die folgenden Schritte auf:
a) Einbringen einer Inertprobe und einer Anregerflüssigkeit in die Probenpräparationskammer,
b) Temperieren der Inertprobe und der Anregerflüssigkeit in der Probenpräparationskammer,
c) Einbringen einer Mischung der Inertprobe und der Anregerflüssigkeit in die Kalorimeterkammer,
d) Erfassen des Wärmeflusses in oder aus der Mischung aus der Inertprobe und der Anregerflüssigkeit,
e) Auswerten des in Schritt d) erfassten Wärmeflusses und Unterscheiden in Fall I) eines positiven Wärmeflusses aus der Mischung der Inertprobe und der Anregerflüssigkeit heraus und in Fall II) eines negativen Wärmeflusses in die Mischung der Inertprobe und der Anregerflüssigkeit hinein,
f) In Fall I) Absenken der Temperatur der Präparationskammertemperierung und/oder Anheben der Temperatur der ersten Kalorimetertemperierung sowie in Fall II) Anheben der Temperatur der Präparationskammertemperierung und/oder Absenken der Temperatur der ersten Kalorimetertemperierung,
g) Wiederholen, bis in Schritt e) kein Wärmefluss im Rahmen der Messgenauigkeit erfasst wird und somit Präparationskammertemperierung und erste Kalorimetertemperierung auf die gleiche absolute Temperatur eingestellt sind.

Das Einbringen in Schritt a) kann von außen erfolgen, wie dieses für Proben normal wäre.

Da es sich aber nur um eine interne Kalibrierung mit einer inerten Probe handelt, kann, wie im Folgenden ausgeführt, die gleiche Probe, bereits als Gemisch, auch wiederverwendet werden. Das Einbringen in Schritt a) kann also auch aus der Kalorimeterkammer erfolgen. Abhängig von der Einbringung kann der anschließende Schritt des Temperierens b) unterschiedlich lang ausfallen. Wird beispielsweise eine Probe wiederverwendet und aus der Kalorimeterkammer in die Probenpräparationskammer eingebracht, so wird die Temperaturdifferenz kleiner sein als eine von außen eingebrachte Probe, sodass hier eine kürzere Zeit für das Temperieren ausreichend ist.

Zwischen dem Temperieren in Schritt b) und dem Einbringen in Schritt c) erfolgt ein Vermischen, sofern die Inertprobe und die Anregerflüssigkeit in Schritt b) getrennt vorliegen. Dieses ist vorteilhaft, da auch durch das Mischen mechanische Arbeit geleistet und dadurch Wärme eingebracht wird. Wird die Probe jedoch "wiederverwendet", so ist diese bereits vermischt, sodass ein erneutes Vermischen nicht notwendig ist. Dennoch kann der Vorgang durchgeführt werden, um den energischen Aspekt des Mischens beizubehalten.

Das Erfassen in Schritt d) erfolgt in einem Zeitfenster von bis zu 60 min. Danach sind alle thermischen Effekte abgeklungen. Daher wird im Stand der Technik die Messung auch nach diesem Zeitfenster begonnen.

Das Auswerten in Schritt e) erfolgt bevorzugt durch Integration über den Zeitverlauf, also die Ermittlung der insgesamt freigesetzten oder aufgenommenen Wärmemenge, so wie dieses auch in der Kalorimetrie sonst üblich ist.

Aus der geflossenen Wärmemenge ergibt sich, ob die Probenpräparationskammer wärmer oder kälter als die erste Kalorimeterkammer ist. Dem entsprechend wird dann in Schritt f) eine Anpassung vorgenommen, bis keine Differenz mehr festzustellen und damit die Probenpräparationskammer und die ersten Kalorimeterkammer die gleiche absolute Temperatur aufweisen.

Der Vorteil dieses Verfahrens ist es, dass dieses automatisiert durchgeführt werden kann, um die absolute Kalibrierung verschiedener Thermoelemente zueinander zu ermöglichen und damit insgesamt einen unverzüglichen Messbeginn im Kalorimeter zu ermöglichen, was wiederum eine Auswertbarkeit auch des Initialpeaks ermöglicht, was wiederum notwendig ist, um eine schnelle Rückmeldung zur Reaktivität im Zementbereich zu bekommen.

In einer weiteren Ausführungsform der Erfindung weist das Wärmeflusskalorimeter eine zweite Kalorimeterkammer auf. Auch die zweite Kalorimeterkammer kann zwei oder mehr Kalorimeterzellen aufweisen. Wie bereits ausgeführt ist eine größere Anzahl, beispielsweise acht Kalorimeterkammern üblich. Die zweite Kalorimeterkammer weist eine zweite Kalorimetertemperierung mit einem zweiten Kalorimeterthermoelement auf. Zunächst wird das Verfahren für die erste Kalorimeterkammer bis zum Erreichen des Schrittes g) durchgeführt. Es wird also zunächst ein Abgleich zwischen der Probenpräparationskammer und der ersten Kalorimeterkammer durchgeführt. Damit ist die Einstellung für die Probenpräparationskammer nicht mehr zu ändern. Daher wird anschließend das Verfahren für die zweite Kalorimeterkammer durchgeführt, wobei in Schritt f) in Fall I) ein Absenken der Temperatur der zweiten Kalorimetertemperierung sowie in Fall II) ein Anheben der Temperatur der zweiten Kalorimetertemperierung erfolgt. Es wird also nur die zweite Kalorimeterkammer angepasst und damit auf das gleiche Temperaturniveau der ersten Kalorimeterkammer und der Probenpräparationskammer gebracht. Anschließend kann das Verfahren entsprechend auch für weitere Kalorimeterkammern durchgeführt werden. Dadurch werden nacheinander alle Kalorimeterkammern auf exakt die gleiche Temperatur gebracht, sodass jede Probe in jeder Kalorimeterkammer gemessen und reproduzierbare Ergebnisse liefert.

In einer weiteren Ausführungsform der Erfindung weist das Wärmeflusskalorimeter eine Probenlagerkammer auf. Die Probenlagerkammer ist im normalen Probenfluss vor der Probenpräparationskammer angeordnet und kann dazu benutzt werden, eine größere Anzahl an Proben einzuschleusen und vorzutemperieren. Die Probenlagerkammer weist eine Probenlagertemperierung und ein Probenlagerthermoelement auf. Zunächst wird das Verfahren für die erste Kalorimeterkammer bis zum Erreichen des Schrittes g) durchgeführt und damit die Probenpräparationskammer auf die endgültige Temperatur eingestellt. Anschließend erfolgt erst ein Einbringen der Inertprobe und der Anregerflüssigkeit in die Probenlagerkammer und dann das Einbringen in die Probenpräparationskammer in Schritt a) aus der Probenlagerkammer. Dann erfolgt in Schritt f) in Fall I) Absenken der Temperatur der Probenlagertemperierung sowie in Fall II) Anheben der Temperatur der Probenlagertemperierung. Damit wird die Probenlagerkammer auf eine Temperatur eingestellt, sodass diese keinen Einfluss auf die Messung hat. Hierbei ist durch die Probenverarbeitung in der Probenpräparationskammer davon auszugehen, dass die absolute Temperatur der Probenlagerkammer nur innerhalb eines gewissen Temperaturbandes liegen muss.

In einer weiteren Ausführungsform der Erfindung wird als Inertprobe Quarz, Korund, Titandioxid oder eine Mischung hieraus gewählt. Diese weisen die beste Vergleichbarkeit zu Klinker oder Zement auf, beispielsweise was Wärmekapazität und andere Eigenschaften angeht, zeigen jedoch keine Reaktion bei der Vermischung mit Wasser oder einer wässrigen Lösung, beispielsweise auch einer schwachen wässrigen Lauge.

In einer weiteren Ausführungsform der Erfindung wird als Anregerflüssigkeit Wasser oder eine wässrige Lösung, insbesondere eine basische wässrige Lösung verwendet.

In einer weiteren Ausführungsform der Erfindung erfolgt die Auswertung in Schritt e) quantitativ und die Anpassung in Schritt f) erfolgt proportional zur quantitativen Abweichung. Somit wird die Anpassung umso schneller erfolgen, je größer die Abweichung ist. Dieses hat den Vorteil, dass die Anzahl der Iterationsschritt reduziert wird.

In einer weiteren Ausführungsform der Erfindung erfolgt die Anpassung in vorgegebenen Schritten. Für jede Anpassung wird der Wärmefluss ermittelt. Mittels linearer Regression wird der Einstellwert für den Nulldurchgang und damit die optimale Temperatureinstellung ermittelt.

In einer weiteren Ausführungsform der Erfindung wird vor einer Wiederholung der Verfahrensschritte nach Schritt f) eine Wartezeit gewartet. Die Wartezeit wird in Abhängigkeit der Höhe der Anpassung gewählt.

In einer weiteren Ausführungsform der Erfindung wird die Mischung aus der Inertprobe und der Anregerflüssigkeit wiederverwendet. Da diese bereits auf der richtigen Temperatur ist, beschleunigt dieses die Durchführung des Verfahrens deutlich.

In einer weiteren Ausführungsform der Erfindung erfolgt das Erfassen in Schritt d) über einen Zeitraum von 2 min bis 40 min jeweils beginnend mit dem Einbringen in Schritt c).

Nachfolgend ist das erfindungsgemäße Verfahren anhand eines in den Zeichnungen dargestellten Ausführungsbeispiels näher erläutert.
Fig. 1 Wärmeflusskalorimeter
Fig. 2 Temperaturabhängigkeit

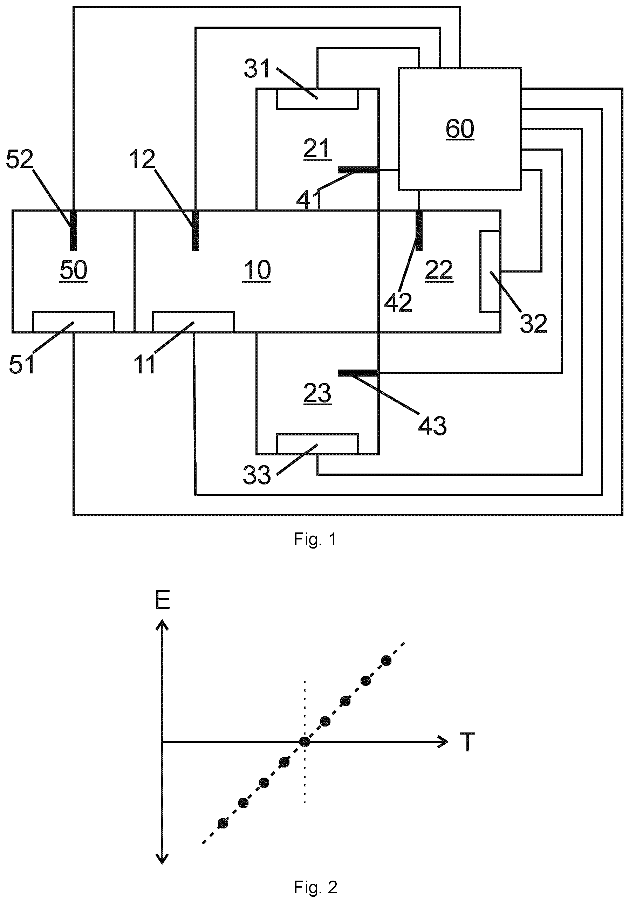

In Fig. 1 ist ein beispielhaftes, schematisiertes Wärmeflusskalorimeter gezeigt. Das Wärmeflusskalorimeter besteht aus einer vorgelagerten Probenlagerkammer 50, einer zentralen Probenpräparationskammer und drei Kalorimeterkammern 21, 22, 23. Die Probenlagerkammer 50 wird über die Probenlagertemperierung 51 temperiert, die Probenpräparationskammer 10 wird über die Präparationskammertemperierung 11 temperiert, die erste Kalorimeterkammer 21 wird über die erste Kalorimetertemperierung 31 temperiert, die zweite Kalorimeterkammer 22 wird über die zweite Kalorimetertemperierung 32 temperiert und die dritte Kalorimeterkammer 23 wird über die dritte Kalorimetertemperierung 33 temperiert. Für die Regelung der Temperierungen 11, 31, 32, 33, 51 sind Thermoelemente 12, 41, 42, 43, 52 vorhanden. Die bereits ausgeführt ist die relative Kalibrierung dieser besonders relevant. Die Probenlagerkammer 50 weist ein Probenlagerthermoelement 52 auf, die Probenpräparationskammer 10 weist ein Präparationskammerthermoelement 12 auf, die erste Kalorimeterkammer 21 weist ein erstes Kalorimeterthermoelement 41 auf, die zweite Kalorimeterkammer 22 weist ein zweites Kalorimeterthermoelement 42 auf und die dritte Kalorimeterkammer 23 weist ein drittes Kalorimeterthermoelement 43 auf. Alle Temperierungen 11, 31, 32, 33, 51 und alle Thermoelemente 12, 41, 42, 43, 52 sind mit einer Steuereinheit 60 verbunden, die die Temperaturen erfasst und die Temperierungen 11, 31, 32, 33, 51 entsprechend steuert.

Fig. 2 zeigt die relative Kalibrierung der Thermoelemente zwischen zwei Bereichen, beispielsweise zwischen der Probenpräparationskammer 10 und der ersten Kalorimeterkammer 21. In diesem Fall wird beispielsweise die Temperatur T in der Probenpräparationskammer 10 variiert, beispielsweise in Schritten von 0,1 K. Für jeden dieser Punkte wird der Wärmefluss E ermittelt. In der Auftragung lässt sich leicht der Nulldurchgang, also die Einstellung finden, in der die zwei Bereiche, beispielsweise die Probenpräparationskammer 10 und die erste Kalorimeterkammer 21, exakt die gleiche Temperatur aufweisen. Dieses ermöglicht dann den unverzüglichen Messbeginn nach Einbringung der Probe und somit die Auswertbarkeit des Initialpeaks.

### Bezugszeichen

- 10: Probenpräparationskammer
- 11: Präparationskammertemperierung
- 12: Präparationskammerthermoelement
- 21: erste Kalorimeterkammer
- 22: zweite Kalorimeterkammer
- 23: dritte Kalorimeterkammer
- 31: erste Kalorimetertemperierung
- 32: zweite Kalorimetertemperierung
- 33: dritte Kalorimetertemperierung
- 41: erste Kalorimeterthermoelement
- 42: zweite Kalorimeterthermoelement
- 43: dritte Kalorimeterthermoelement
- 50: Probenlagerkammer
- 51: Probenlagertemperierung
- 52: Probenlagerthermoelement
- 60: Steuereinheit

## Patentansprüche

1. Verfahren zur Kalibrierung eines Wärmeflusskalorimeters mit wenigstens einer Probenpräparationskammer (10) und wenigstens einer ersten Kalorimeterkammer (21), wobei die Probenpräparationskammer (10) eine Präparationskammertemperierung (11) und ein Präparationskammerthermoelement (12) aufweist, wobei die erste Kalorimeterkammer (21) eine erste Kalorimetertemperierung (31) und ein erstes Kalorimeterthermoelement (41) aufweist, wobei das Wärmeflusskalorimeter eine Steuereinheit (60) aufweist, wobei die Steuereinheit (60) mit dem Präparationskammerthermoelement (12) und dem ersten Kalorimeterthermoelement (41) zur Übertragung der erfassten Temperaturen an die Steuereinheit (60) verbunden ist, wobei die Steuereinheit (60) mit der Präparationskammertemperierung (11) und der ersten Kalorimetertemperierung (31) zur Ansteuerung verbunden ist, wobei das Verfahren die folgenden Schritte aufweist:
a) Einbringen einer Inertprobe und einer Anregerflüssigkeit in die Probenpräparationskammer (10),
b) Temperieren der Inertprobe und der Anregerflüssigkeit in der Probenpräparationskammer (10),
c) Einbringen einer Mischung der Inertprobe und der Anregerflüssigkeit in die Kalorimeterkammer (21),
d) Erfassen des Wärmeflusses in oder aus der Mischung aus der Inertprobe und der Anregerflüssigkeit,
e) Auswerten des in Schritt d) erfassten Wärmeflusses und Unterscheiden in Fall I) eines positiven Wärmeflusses aus der Mischung der Inertprobe und der Anregerflüssigkeit heraus und in Fall II) eines negativen Wärmeflusses in die Mischung der Inertprobe und der Anregerflüssigkeit hinein,
f) In Fall I) Absenken der Temperatur der Präparationskammertemperierung (11) und/oder Anheben der Temperatur der ersten Kalorimetertemperierung (31) sowie in Fall II) Anheben der Temperatur der Präparationskammertemperierung (11) und/oder Absenken der Temperatur der ersten Kalorimetertemperierung (31),
g) Wiederholen, bis in Schritt e) kein Wärmefluss im Rahmen der Messgenauigkeit erfasst wird und somit Präparationskammertemperierung (11) und erste Kalorimetertemperierung (31) auf die gleiche absolute Temperatur eingestellt sind.

2. Verfahren nach Anspruch 1, **dadurch gekennzeichnet, dass** das Wärmeflusskalorimeter eine zweite Kalorimeterkammer (22) aufweist, wobei die zweite Kalorimeterkammer (22) eine zweite Kalorimetertemperierung (32) und ein zweites Kalorimeterthermoelement (42) aufweist, wobei zunächst das Verfahren für die erste Kalorimeterkammer (21) bis zum Erreichen des Schrittes g) durchgeführt wird, wobei anschließend das Verfahren für die zweite Kalorimeterkammer (22) durchgeführt wird, wobei in Schritt f) in Fall I) ein Absenken der Temperatur der zweiten Kalorimetertemperierung (32) sowie in Fall II) ein Anheben der Temperatur der zweiten Kalorimetertemperierung (32) erfolgt.

3. Verfahren nach einem der vorstehenden Ansprüche, **dadurch gekennzeichnet, dass** das Wärmeflusskalorimeter eine Probenlagerkammer (50) aufweist, wobei die Probenlagerkammer (50) eine Probenlagertemperierung (51) und ein Probenlagerthermoelement (52) aufweist, wobei zunächst das Verfahren für die erste Kalorimeterkammer (21) bis zum Erreichen des Schrittes g) durchgeführt wird, wobei anschließend erst ein Einbringen der Inertprobe und der Anregerflüssigkeit in die Probenlagerkammer (50) erfolgt und dann das Einbringen in die Probenpräparationskammer (10) in Schritt a) aus der Probenlagerkammer (50) erfolgt, wobei dann in Schritt f) in Fall I) Absenken der Temperatur der Probenlagertemperierung (51) sowie in Fall II) Anheben der Temperatur der Probenlagertemperierung (51) erfolgt.

4. Verfahren nach einem der vorstehenden Ansprüche, **dadurch gekennzeichnet, dass** als Inertprobe Quarz, Korund, Titandioxid oder eine Mischung hieraus gewählt wird.

5. Verfahren nach einem der vorstehenden Ansprüche, **dadurch gekennzeichnet, dass** als Anregerflüssigkeit Wasser oder eine wässrige Lösung, insbesondere eine basische wässrige Lösung verwendet wird.

6. Verfahren nach einem der vorstehenden Ansprüche, **dadurch gekennzeichnet, dass** die Auswertung in Schritt e) quantitativ erfolgt und dass die Anpassung in Schritt f) proportional zur quantitativen Abweichung erfolgt.

7. Verfahren nach einem der vorstehenden Ansprüche, **dadurch gekennzeichnet, dass** vor einer Wiederholung der Verfahrensschritte nach Schritt f) eine Wartezeit gewartet wird, wobei die Wartezeit in Abhängigkeit der Höhe der Anpassung gewählt wird.

8. Verfahren nach einem der vorstehenden Ansprüche, **dadurch gekennzeichnet, dass** die Mischung aus der Inertprobe und der Anregerflüssigkeit wiederverwendet wird.

9. Verfahren nach einem der vorstehenden Ansprüche, **dadurch gekennzeichnet, dass** das Erfassen in Schritt d) über einen Zeitraum von 2 min bis 40 min nach dem Einbringen in Schritt c) erfolgt.

## Claims

1. A method for the calibration of a heat flow calorimeter with at least one sample preparation chamber (10) and at least one first calorimeter chamber (21), wherein the sample preparation chamber (10) comprises a preparation chamber temperature control (11) and a preparation chamber thermocouple (12), wherein the first calorimeter chamber (21) comprises a first calorimeter temperature control (31) and a first calorimeter thermocouple (41), wherein the heat flow calorimeter comprises a control unit (60), wherein the control unit (60) is connected to the preparation chamber thermocouple (12) and the first calorimeter thermocouple (41) for the transmission of the recorded temperatures to the control unit (60), wherein the control unit (60) is connected to the preparation chamber temperature control (11) and the first calorimeter temperature control (31) for control, wherein the method comprises the following steps:
a) introducing an inert sample and an excitation liquid into the sample preparation chamber (10),
b) temperature-controlling the inert sample and the excitation liquid in the sample preparation chamber (10),
c) introducing a mixture of the inert sample and the excitation liquid into the calorimeter chamber (21),
d) recording the heat flow into or out of the mixture of the inert sample and the excitation liquid,
e) evaluating the heat flow recorded in step d) and distinguishing into case I) of a positive heat flow out of the mixture of the inert sample and the excitation liquid and into case II) of a negative heat flow into the mixture of the inert sample and the excitation liquid,
f) in case I) lowering the temperature of the preparation chamber temperature control (11) and/or raising the temperature of the first calorimeter temperature control (31) and in case II) raising the temperature of the preparation chamber temperature control (11) and/or lowering the temperature of the first calorimeter temperature control (31),
g) repeating until in step e) no heat flow is recorded within the measurement accuracy and thus the preparation chamber temperature control (11) and the first calorimeter temperature control (31) are set to the same absolute temperature.

2. The method according to claim 1, **characterized in that** the heat flow calorimeter comprises a second calorimeter chamber (22), wherein the second calorimeter chamber (22) comprises a second calorimeter temperature control (32) and a second calorimeter thermocouple (42), wherein first the method for the first calorimeter chamber (21) is carried out until reaching step g), wherein subsequently the method for the second calorimeter chamber (22) is carried out, wherein in step f) in case I) a lowering of the temperature of the second calorimeter temperature control (32) and in case II) a raising of the temperature of the second calorimeter temperature control (32) takes place.

3. The method according to one of the preceding claims, **characterized in that** the heat flow calorimeter comprises a sample storage chamber (50), wherein the sample storage chamber (50) comprises a sample storage temperature control (51) and a sample storage thermocouple (52), wherein first the method for the first calorimeter chamber (21) is carried out until reaching step g), wherein subsequently only then an introduction of the inert sample and the excitation liquid into the sample storage chamber (50) takes place and then the introduction into the sample preparation chamber (10) in step a) takes place from the sample storage chamber (50), wherein then in step f) in case I) lowering the temperature of the sample storage temperature control (51) and in case II) raising the temperature of the sample storage temperature control (51) takes place.

4. The method according to one of the preceding claims, **characterized in that** quartz, corundum, titanium dioxide or a mixture thereof is selected as inert sample.

5. The method according to one of the preceding claims, **characterized in that** water or an aqueous solution, in particular a basic aqueous solution, is used as excitation liquid.

6. The method according to one of the preceding claims, **characterized in that** the evaluation in step e) is carried out quantitatively and that the adjustment in step f) is carried out proportionally to the quantitative deviation.

7. The method according to one of the preceding claims, **characterized in that** before a repetition of the method steps after step f) a waiting time is waited, wherein the waiting time is selected as a function of the magnitude of the adjustment.

8. The method according to one of the preceding claims, **characterized in that** the mixture of the inert sample and the excitation liquid is reused.

9. The method according to one of the preceding claims, **characterized in that** the recording in step d) takes place over a period of 2 min to 40 min after the introduction in step c).

## Revendications

1. Un procédé pour l'étalonnage d'un calorimètre à flux de chaleur avec au moins une chambre de préparation d'échantillon (10) et au moins une première chambre de calorimètre (21), dans lequel la chambre de préparation d'échantillon (10) comprend un contrôle de température de la chambre de préparation (11) et un thermocouple de la chambre de préparation (12), dans lequel la première chambre de calorimètre (21) comprend un premier contrôle de température du calorimètre (31) et un premier thermocouple du calorimètre (41), dans lequel le calorimètre à flux de chaleur comprend une unité de commande (60), dans lequel l'unité de commande (60) est reliée au thermocouple de la chambre de préparation (12) et au premier thermocouple du calorimètre (41) pour la transmission des températures enregistrées à l'unité de commande (60), dans lequel l'unité de commande (60) est reliée au contrôle de température de la chambre de préparation (11) et au premier contrôle de température du calorimètre (31) pour la commande, dans lequel le procédé comprend les étapes suivantes:
a) introduction d'un échantillon inerte et d'un liquide d'excitation dans la chambre de préparation d'échantillon (10),
b) contrôle de température de l'échantillon inerte et du liquide d'excitation dans la chambre de préparation d'échantillon (10),
c) introduction d'un mélange de l'échantillon inerte et du liquide d'excitation dans la chambre de calorimètre (21),
d) enregistrement du flux de chaleur dans ou hors du mélange de l'échantillon inerte et du liquide d'excitation,
e) évaluation du flux de chaleur enregistré à l'étape d) et distinction en cas I) d'un flux de chaleur positif hors du mélange de l'échantillon inerte et du liquide d'excitation et en cas II) d'un flux de chaleur négatif dans le mélange de l'échantillon inerte et du liquide d'excitation,
f) en cas I) abaissement de la température du contrôle de température de la chambre de préparation (11) et/ou élévation de la température du premier contrôle de température du calorimètre (31) et en cas II) élévation de la température du contrôle de température de la chambre de préparation (11) et/ou abaissement de la température du premier contrôle de température du calorimètre (31),
g) répétition jusqu'à ce qu'à l'étape e) aucun flux de chaleur ne soit enregistré dans le cadre de la précision de mesure et ainsi le contrôle de température de la chambre de préparation (11) et le premier contrôle de température du calorimètre (31) soient réglés à la même température absolue.

2. Le procédé selon la revendication 1, **caractérisé en ce que** le calorimètre à flux de chaleur comprend une deuxième chambre de calorimètre (22), dans laquelle la deuxième chambre de calorimètre (22) comprend un deuxième contrôle de température du calorimètre (32) et un deuxième thermocouple du calorimètre (42), dans lequel d'abord le procédé pour la première chambre de calorimètre (21) est effectué jusqu'à atteindre l'étape g), dans lequel ensuite le procédé pour la deuxième chambre de calorimètre (22) est effectué, dans lequel à l'étape f) en cas I) un abaissement de la température du deuxième contrôle de température du calorimètre (32) et en cas II) une élévation de la température du deuxième contrôle de température du calorimètre (32) a lieu.

3. Le procédé selon l'une des revendications précédentes, **caractérisé en ce que** le calorimètre à flux de chaleur comprend une chambre de stockage d'échantillon (50), dans laquelle la chambre de stockage d'échantillon (50) comprend un contrôle de température de stockage d'échantillon (51) et un thermocouple de stockage d'échantillon (52), dans lequel d'abord le procédé pour la première chambre de calorimètre (21) est effectué jusqu'à atteindre l'étape g), dans lequel ensuite seulement une introduction de l'échantillon inerte et du liquide d'excitation dans la chambre de stockage d'échantillon (50) a lieu et ensuite l'introduction dans la chambre de préparation d'échantillon (10) à l'étape a) a lieu à partir de la chambre de stockage d'échantillon (50), dans lequel ensuite à l'étape f) en cas I) abaissement de la température du contrôle de température de stockage d'échantillon (51) et en cas II) élévation de la température du contrôle de température de stockage d'échantillon (51) a lieu.

4. Le procédé selon l'une des revendications précédentes, **caractérisé en ce que** le quartz, le corindon, le dioxyde de titane ou un mélange de ceux-ci est sélectionné comme échantillon inerte.

5. Le procédé selon l'une des revendications précédentes, **caractérisé en ce que** de l'eau ou une solution aqueuse, en particulier une solution aqueuse basique, est utilisée comme liquide d'excitation.

6. Le procédé selon l'une des revendications précédentes, **caractérisé en ce que** l'évaluation à l'étape e) est effectuée quantitativement et que l'ajustement à l'étape f) est effectué proportionnellement à la déviation quantitative.

7. Le procédé selon l'une des revendications précédentes, **caractérisé en ce qu'**avant une répétition des étapes du procédé après l'étape f) un temps d'attente est attendu, dans lequel le temps d'attente est sélectionné en fonction de l'ampleur de l'ajustement.

8. Le procédé selon l'une des revendications précédentes, **caractérisé en ce que** le mélange de l'échantillon inerte et du liquide d'excitation est réutilisé.

9. Le procédé selon l'une des revendications précédentes, **caractérisé en ce que** l'enregistrement à l'étape d) a lieu sur une période de 2 min à 40 min après l'introduction à l'étape c).
